# EUROPEAN PATENT SPECIFICATION

(11) **EP 3 435 968 B1**
(45) Date of publication and mention of the grant of the patent: **25.09.2019**
(21) Application number: 17707922.5
(22) Date of filing: 06.03.2017
(51) Int. Cl.: A61K 8/46, A61Q 5/02, A61Q 13/00, A61K 8/73, A61Q 19/10, A61K 8/11

(54) **PERSONAL CLEANSING COMPOSITION**
REINIGUNGSZUSAMMENSETZUNG FÜR DEN PERSONLICHEN GEBRAUCH
COMPOSITION NETTOYANTE CORPORELLE

(30) Priority: 01.04.2016 EP 16163563
(43) Date of publication of application: 06.02.2019
(73) Proprietor: Unilever PLC, London, Greater London EC4Y 0DY (GB); Unilever N.V., 3013 AL Rotterdam (NL)
(72) Inventor: ALONSO, Coralie, Claudine, Bebington Wirral Merseyside CH63 3JW (GB); CLARKSON, Heather, Bebington Wirral Merseyside CH63 3JW (GB); SHAW, Neil, Scott, Bebington Wirral Merseyside CH63 3JW (GB)
(74) Representative: Chisem, Janet
(86) International application number: PCT/EP2017/055152
(87) International publication number: WO 2017/167552

(56) References cited:
- WO-A1-2004/043413
- WO-A1-2004/043414
- WO-A1-2007/065537
- WO-A1-2009/100464

## Description

### Field of the Invention

The present invention relates to personal care cleansing compositions such as liquid soaps, body washes and shampoos.

### Background and Prior Art

In personal cleansing compositions such as liquid soaps, body washes and shampoos, the deposition and delivery of benefit agents are often key drivers of product performance. For example, many of the shampoo products in the market today work to deliver benefits to hair by depositing benefit agents such as fragrance materials, silicones, dyes, and anti-dandruff agents onto the hair during washing.

Various technologies have been employed to enhance the delivery of benefit agents at the desired time. One widely used technology is encapsulation of the benefit agent in a protective coating such as a polymeric material. The polymeric material may protect the benefit agent, such as a fragrance material, from evaporation, reaction, oxidation or otherwise dissipating prior to use.

However, maximizing encapsulate deposition during cleansing is a difficult task since most personal cleansing compositions were designed to carry away particulates from the skin or hair. When encapsulates are washed away, relatively high levels of encapsulated benefit agents may be needed in the composition to deliver the consumer desired benefit.

WO 2009/100464 discloses a composition comprising a coated microparticulate, which comprises a benefit agent and is coated with a Type-1 polymer comprising a cationic polymer with a cationic atom content greater than about 3 wt % and a wt average MWt less than about 800,000 Da; and a Type-2 polymer comprising a cationic polymer with a cationic atom content of less than about 3 wt % and a wt average MWt greater than about 1, 000,000 Da.

WO2007/065537 discloses an aqueous shampoo composition comprising: (i) one or more anionic cleansing surfactants; (ii) discrete, dispersed droplets of a water-insoluble conditioning agent with a mean droplet diameter (D_{3,2}) of 4 micrometres or less; (iii) one or more cationic polymers (A) selected from cationically modified acrylamide polymers having a cationic charge density at pH7 of less than 1.0 meq per gram, cationically modified celluloses and mixtures thereof. and (iv) one or more cationic polymers (B) selected from cationically modified acrylamide polymers having a cationic charge density at pH7 of greater than 1.0 meq per gram, cationically modified polygalactomannans, and mixtures thereof, wherein the composition comprises a cationic polymer other than a cationically modified acrylamide polymer. Silicones are preferred and exemplified as water insoluble conditioning agent.

Despite the prior art there is a need for a personal cleansing composition that provides an increased deposition of encapsulated benefit agents onto the hair or skin, without impairing other product attributes such as rheology, sensory and conditioning performance.

The present invention addresses this problem.

### Definition of the Invention

In a first aspect, the present invention provides a personal cleansing composition comprising, in an aqueous continuous phase:
(i) from 5 to 30% by weight of one or more anionic cleansing surfactants;
(ii) microcapsules in which a core comprising benefit agent is encapsulated in a polymeric shell, and in which the benefit agent of the core of the microcapsules (ii) is selected from perfumes,
(iii) a fragrance, and
(iv) a combination of cationic polymers comprising:
   (a) at least one cationic polygalactomannan having a mean charge density at pH 7 of less than 1.2 meq per gram, preferably from 0.5 to 1.1; and
   (b) at least one cationic polygalactomannan having a mean charge density at pH 7 at least 1.2 meq per gram, preferably from 1.2 to 3, more preferably from 1.2 to 2.

### Detailed Description and Preferred Embodiments

All molecular weights as used herein are weight average molecular weights, unless otherwise specified.

By "aqueous continuous phase" is meant a continuous phase which has water as its basis.

Suitably, the composition of the invention will comprise from about 50 to about 90%, preferably from about 55 to about 85%, more preferably from about 60 to about 85%, most preferably from about 65 to about 83% water (by weight based on the total weight of the composition).

Typical anionic cleansing surfactants (i) for use in the invention include those surface active agents which contain an organic hydrophobic group with from 8 to 14 carbon atoms, preferably from 10 to 14 carbon atoms in their molecular structure; and at least one water-solubilising group which is preferably selected from sulphate, sulphonate, sarcosinate and isethionate.

Specific examples of such anionic cleansing surfactants include ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

A preferred class of anionic cleansing surfactants for use in the invention are alkyl ether sulphates of general formula:

R-O-(CH₂CH₂-O)ₙ-SO₃⁻M⁺

in which R is a straight or branched chain alkyl group having 10 to 14 carbon atoms, n is a number that represents the average degree of ethoxylation and ranges from 1 to 5, preferably from 1 to 3, and M is a alkali metal, ammonium or alkanolammonium cation, preferably sodium, potassium, monoethanolammonium or triethanolammonium, or a mixture thereof.

Specific examples of such preferred anionic surfactants include the sodium, potassium, ammonium or ethanolamine salts of C₁₀ to C₁₂ alkyl sulphates and C₁₀ to C₁₂ alkyl ether sulphates (for example sodium lauryl ether sulphate),

Mixtures of any of the above described materials may also be used.

In a typical composition according to the invention the level of anionic cleansing surfactant ranges from 5 to 30 wt %, by weight based on the total weight of the composition.

The aqueous continuous phase of the composition according to the invention preferably also includes one or more amphoteric surfactants, in addition to the anionic cleansing surfactant described above. Suitable amphoteric surfactants are betaines, such as those having the general formula R(CH₃)₂N⁺CH₂COO⁻, where R is an alkyl or alkylamidoalkyl group, the alkyl group preferably having 10 to 16 carbon atoms. Particularly suitable betaines are oleyl betaine, caprylamidopropyl betaine, lauramidopropyl betaine, isostearylamidopropyl betaine, and cocoamidopropyl betaine. Cocoamidopropyl betaine is particularly preferred.

When included, the total level of amphoteric surfactant is preferably from 0.1 to 10%, more preferably from 0.5 to 5%, and most preferably from 1 to 3% by weight based on the total weight of the hair cleansing composition).

The compositions of the invention preferably comprise dispersed droplets of conditioning agent with a mean diameter (D3,2) of 4 micrometres or less.

The preferred amount of these dispersed droplets is from 0.1 to 10% by weight of the total composition.

The preferred dispersed conditioning agent is an emulsified silicone.

Droplets of emulsified silicone for inclusion in the composition of the invention typically have a mean droplet diameter (D3,2) of 2 micrometres or less. Preferably the mean droplet diameter (D3,2) is 1 micrometre or less, more preferably 0.5 micrometre or less, and most preferably 0.25 micrometre or less.

A suitable method for measuring the mean droplet diameter (D3,2) is by laser light scattering using an instrument such as a Malvern Mastersizer.

Suitable silicones for use in the invention include polydiorganosiloxanes, in particular polydimethylsiloxanes (dimethicones), polydimethyl siloxanes having hydroxyl end groups (dimethiconols), and amino-functional polydimethylsiloxanes (amodimethicones).

Such silicones are preferably non-volatile (with vapour pressure of less than 1000 Pa at 25°C), and preferably have a molecular weight of greater than 100,000, more preferably greater than 250,000.

Such silicones preferably have a kinematic viscosity of greater than 50,000 cS (mm².s⁻¹) and more preferably a kinematic viscosity of greater than 500,000 cS (mm².s⁻¹). Silicone kinematic viscosities in the context of this invention are measured at 25°C and can be measured by means of a glass capillary viscometer as set out further in Dow Corning Corporate Test Method CTM004 July 20, 1970.

Suitable silicones for use in the invention are available as pre-formed silicone emulsions from suppliers such as Dow Corning and GE Silicones. The use of such pre-formed silicone emulsions is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Pre-formed silicone emulsions having a mean droplet diameter (D3,2) of less than 0.15 micrometres are generally termed microemulsions.

Examples of suitable pre-formed silicone emulsions include emulsions DC2-1766, DC2-1784, DC-1785, DC-1786, DC-1788, DC-1310, DC-7123 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Mixtures of any of the above described silicone emulsions may also be used.

In a typical composition according to the invention the level of silicone (*per se* as active ingredient) will generally range from 1 to 8%, and preferably ranges from 2 to 7.5% by weight based on the total weight of the composition.

The composition of the invention may suitably include at least one inorganic electrolyte. The inorganic electrolyte may be used to help provide viscosity to the composition.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

Suitable inorganic electrolytes include metal chlorides (such as sodium chloride, potassium chloride, calcium chloride, magnesium chloride, zinc chloride, ferric chloride and aluminium chloride) and metal sulphates (such as sodium sulphate and magnesium sulphate).

Examples of preferred inorganic electrolytes for use in the invention include sodium chloride, potassium chloride, magnesium sulphate and mixtures thereof.

The composition of the invention comprises microcapsules (iii) in which a core comprising benefit agent is encapsulated in a polymeric shell.

The microcapsules are preferably present in an amount of from 0.1 to 5% by weight of the total composition.

The term "benefit agent" in the context of this invention includes materials which can provide a benefit to the hair and/or the scalp and/or the skin (preferably the hair and/or the scalp) as well as those materials which are beneficially incorporated into personal cleansing compositions, such as aesthetic agents.

The benefit agent of the core of the microcapsule is selected from perfumes.

A perfume normally consists of a mixture of a number of perfume materials, each of which has an odour or fragrance. The number of perfume materials in a perfume is typically 10 or more. The range of fragrant materials used in perfumery is very wide; the materials come from a variety of chemical classes, but in general are water-insoluble oils. In many instances, the molecular weight of a perfume material is in excess of 150, but does not exceed 300.

Examples of perfume materials for use in the invention include geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopyl acetate, 2-phenyl-ethanol, 2-penylethyl acetate, benzyl alcohol, benzyl acetate, benzyl salicylate, styrallyl acetate, benzyl benzoate, amyl salicylate, dimethylbenzyl-carbinol, trichloromethylphenyl-carbinyl acetate, p-tert-butylcyclohexyl acetate, isononyl acetate, vetiveryl acetate, vetiverol, α-hexylcinnamaldehyde, 2-methyl-3-p-tert-butylpheyl)propanal, 2-methyl-3-(p-isopropylphenyl)propanal, 2-(p-tert-butylpheyl)propanal, 2,4-dimethyl-cyclohex-3-enyl-carboxaldehyde, tricyclodecenyl acetate, tricyclodecenyl propionate,4-(4-hydroxy-4-methylpentyl)-3-cyclohexenecarboxyaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethyl-acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate and mixtures thereof..

Optional further materials which may be included in the core of the microcapsule include dyes, pigments and preservatives.

The polymeric shell of the microcapsule may be prepared using methods known to those skilled in the art such as coacervation, interfacial polymerisation and polycondensation.

The process of coacervation typically involves encapsulation of a generally water-insoluble material by the precipitation of colloidal material(s) onto the surface of droplets of the material. Coacervation may be simple e.g. using one colloid such as gelatin, or complex where two or possibly more colloids of opposite charge, such as gelatin and gum arabic or gelatin and carboxymethyl cellulose, are used under carefully controlled conditions of pH, temperature and concentration.

Interfacial polymerisation produces encapsulated shells from the reaction of at least one oil-soluble wall forming material present in the oil phase with at least one water-soluble wall forming material present in the aqueous phase. A polymerisation reaction between the two wall-forming materials occurs resulting in the formation of covalent bonds at the interface of the oil and aqueous phases to form the capsule wall. An example of a shell capsule produced by this method is a polyurethane capsule.

Polycondensation involves forming a dispersion or emulsion of water-insoluble material (e.g. perfume) in an aqueous solution of precondensate of polymeric materials under appropriate conditions of agitation to produce capsules of a desired size, and adjusting the reaction conditions to cause condensation of the precondensate by acid catalysis, resulting in the condensate separating from solution and surrounding the dispersed water-insoluble material to produce a coherent film and the desired microcapsules.

A preferred method for forming microcapsules for use in the invention is polycondensation, typically to produce aminoplast encapsulates. Aminoplast resins are the reaction products of one or more amines with one or more aldehydes. Examples of suitable amines include urea, thiourea, melamine and its derivatives, benzoguanamine and acetoguanamine and combinations of amines.

Preferably the polymeric shell of the microcapsule is an aminoplast resin selected from melamine formaldehyde, urea formaldehyde, melamine glyoxal and polyurea formed by reaction of polyisocyanates and polyamines. The most preferred polymeric shell is selected from melamine glyoxal and polyurea.

Preferably, the microcapsules are activated by shear; that is to say they are broken by shear to release the contents.

A particularly preferred microcapsule has a melamine glyoxyl shell, prepared as described in WO2013/068255 and WO2011/161618 and available from Firmenich SA.

Advantageously the polymeric shell comprises at most 20 wt% of the weight of the microcapsules.

By modifying process conditions microcapsules of a desired size can be produced in known manner. The microcapsules typically have a mean diameter in the range 1 to 500 microns, preferably 1 to 300 microns, more preferably 1 to 50 microns and most preferably 1 to 10 microns. If necessary, the microcapsules as initially produced may be filtered or screened to produce a product of greater size uniformity.

In a typical composition according to the invention the level of microcapsules will generally range from 0.2 to 2%, and preferably ranges from 0.5 to 1.5% by weight based on the total weight of the composition.

The composition of the invention comprises, *inter alia,* a combination of cationic polymers comprising:
(a) at least one cationic polygalactomannan having a mean charge density at pH 7 of less than 1.2 meq per gram, preferably from 0.5 to 1.1; and
(b) at least one cationic polygalactomannan having a mean charge density at pH 7 at least 1.2 meq per gram, preferably from 1.2 to 3, more preferably from 1.2 to 2

The term "charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain.

The polygalactomannans are polysaccharides composed principally of galactose and mannose units and are usually found in the endosperm of leguminous seeds, such as guar, locust bean, honey locust, flame tree, and the like. Guar flour is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is therefore one to two.

Suitable cationic polygalactomannans (a) for use in the invention include polygalactomannans, such as guars, and polygalactomannan derivatives, such as hydroxyalkyl guars (for example hydroxyethyl guars or hydroxypropyl guars), that have been cationically modified by chemical reaction with one or more derivatizing agents.

Derivatizing agents typically contain a reactive functional group, such as an epoxy group, a halide group, an ester group, an anhydride group or an ethylenically unsaturated group, and at least one cationic group such as a cationic nitrogen group, more typically a quaternary ammonium group. The derivatization reaction typically introduces lateral cationic groups on the polygalactomannan backbone, generally linked via ether bonds in which the oxygen atom corresponds to hydroxyl groups on the polygalactomannan backbone which have reacted.

Preferred cationic polygalactomannans (a) for use in the invention include guar hydroxypropyltrimethylammonium chlorides.

Guar hydroxypropyltrimethylammonium chlorides for use in the invention are generally comprised of a nonionic guar gum backbone that is functionalized with ether-linked 2-hydroxypropyltrimethylammonium chloride groups, and are typically prepared by the reaction of guar gum with N-(3-chloro-2-hydroxypropyl) trimethylammonium chloride. Cationic polygalactomannans for use in the invention (preferably guar hydroxypropyltrimethylammonium chlorides) generally have an average molecular weight (weight average molecular mass (Mw) determined by size exclusion chromatography) in the range 500,000 to 3 million g/mol, more preferably 800,000 to 2.5 million g/mol.

The cationic charge density of the polymer is suitably determined via the Kjeldahl method as described in the US Pharmacopoeia under chemical tests for nitrogen determination.

Specific examples of preferred cationic polygalactomannans (a) are guar hydroxypropyltrimonium chlorides having a cationic charge density from 0.5 to 1.1 meq/g.

Specific examples of preferred cationic polygalactomannans (b) are guar hydroxypropyltrimonium chlorides having a cationic charge density from 1.2 to 2 meq per gram.

Specific examples of preferred mixtures of cationic polygalactomannans are mixtures of guar hydroxypropyltrimonium chlorides in which one has a cationic charge density from 0.5 to 1.1 meq/g, and one has a cationic charge density from 1.2 to 2 meq per gram.

Cationic polygalactomannans (a) for use in the invention are commercially available from Rhodia as JAGUAR ® C13S, JAGUAR ® C14.

A cationic polygalactomannan (b) for use in the invention is commercially available from Rhodia as JAGUAR ® C17.

A preferred cationic polygalactomannan (a) is selected from guar hydroxypropyltrimonium chlorides having an average molecular weight in the range 800,000 to 2.5 million g/mol and a charge density ranging from 0.5 to 1.1 meq/g.

A preferred cationic polygalactomannan (b) is selected from guar hydroxypropyltrimonium chlorides having an average molecular weight in the range 800,000 to 2.5 million g/mol and a charge density ranging from 1.2 to 2 meq/g.

In a typical composition according to the invention the total level of cationic polygalactomannans will generally range from 0.05 to 0.5%, and preferably ranges from 0.1 to 0.3%, more preferably from 0.15 to 0.25 % by weight based on the total weight of the composition.

A composition of the invention contains further ingredients to enhance performance and/or consumer acceptability. This ingredient is a fragrance. This ingredient is included at a level of up to 5% by weight based on the total weight of the composition.

The pH of the composition of the invention preferably ranges from 4 to 7, more preferably from 5.5 to 6.5.

### Mode of Use

The composition of the invention is primarily intended for topical application to the body, preferably the hair and scalp.

Most preferably the composition of the invention is topically applied to the hair and then massaged into the hair and scalp. The composition is then rinsed off the hair and scalp with water prior to drying the hair.

The invention will be further illustrated by the following, non-limiting Examples, in which all percentages quoted are by weight based on total weight unless otherwise stated.

### EXAMPLES

Three shampoo compositions were prepared.

Enacps (melamine glyoxyl encaps, purchased from Firmenich) containing an oil soluble dye, Hostasol, which fluoresces were added by post dosing to each of the bases at a level of 0.4 wt % by total weight of the composition, as shown in Table 1 below.

**Table 1: summary of shampoo compositions SH1, SH2 and SH3**

| Shampoo | Base | Encap |
|---|---|---|
| SH1 | Commercially available shampoo, Pantene Pro-V Total Damage Care 10, available in Indonesia. Comprises guar hydroxypropyltrimonium chloride. | 0.4 wt % |
| SH2 | Shampoo composition, shown below | 0.4 wt % |
| SH3 | Shampoo composition with polygalactomannan blend, shown below, in accordance with the invention. | 0.4 wt % |

**Table 2: Composition of SH2:**

| **Ingredient** | **Amount wt %)** |
|---|---|
| Sodium laureth sulfate (2EO) | 17.0 |
| Cocamidopropyl betaine | 5.3 |
| Carbomer | 0.4 |
| Perfume | 0.7 |
| Silicone (DOW CORNING® 1788 Emulsion) | 5.0 |
| Encapsulated perfume (ex Firmenich) | 0.4 |
| Water, minors | To 100 |

**Table 3: Composition of SH3:**

| **Ingredient** | **Amount (wt %)** |
|---|---|
| Sodium laureth sulfate (2EO) | 17.0 |
| Cocamidopropyl betaine | 5.3 |
| Carbomer | 0.4 |
| JAGUAR® C14S | 0.15 |
| JAGUAR® C17 | 0.05 |
| Perfume | 0.7 |
| Silicone (DOW CORNING® 1788 Emulsion) | 5.0 |
| Encapsulated perfume (ex Firmenich) | 0.4 |
| Water, minors | To 100 |

### Example 1 : Deposition of capsules on hair from SH1. SH2 and SH3

To measure the deposition of capsules onto hair, the following method was used:
2 inch, 250 mg, switches of virgin Caucasian hair were used.

The hair was first washed with 0.1 g shampoo composition (SH1, SH2 or SH3), per g hair. Tresses were lathered for 30 s and then rinsed in warm water (35 °C - 40 °C) for 30s.

The washed hair was then subjected to an ethanol extraction. The whole 2 inch hair switch was cut into a vial containing 2ml ethanol used to extract the fluorescer deposited in the encap. Hair samples were rolled for one hour during solvent extraction. The concentration of fluouescer in 200 microlitre aliquots of ethanolic solutions obtained as above were determined using appropriate calibration standards.

The extracted samples were placed into a 96-well plate and analysed by fluorescence spectrometry on a Varioskan Fluorescence detector to determine the level of deposition of the microcapsules onto the hair. An excitation wavelength of 450nm and an emission wavelength of 520nm were used.

The results are given in Table 2 below.

**Table 2: Level of deposition of microcapsules on hair, from SH1, SH2 and SH3**

| Composition | Deposition Efficiency |
|---|---|
| | (%) |
| SH1 | 14.7 |
| SH2 | 17.8 |
| SH3 | 23.0 |

It will be seen that deposition is greatest in SH3, in accordance with the invention. This is in contrast to SH1, which contains the same amount of encaps also in combination with guar hydroxypropyltrimonium chloride polymer.

### Example 2: Perfume Intensity on hair treated with SH1, SH2 and SH3

Perfume intensity of hair treated with SH1, SH2 and SH3 was evaluated by a fragrance expert before and after combing the switches.

The hair switches were first washed with the composition following wash protocol given above. 7" switches were used. The switches are then left to dry overnight. The perfume intensity was measured 24 hours after washing.

The perfume intensity results are reported in Table 3.

**Table 3: Perfume intensity of hair treated with SH1, SH2 and SH3**

| Composition | Measurement | Perfume intensity |
|---|---|---|
| SH1 | Before combing | 5 |
| | After combing | 5.5 |
| SH2 | Before combing | 4.5 |
| | After combing | 6 |
| SH3 | Before combing | 6 |
| | After combing | 7.5 |

It will be seen that perfume intensity is greatest in hair treated with SH3, in accordance with the invention.

### Example 3: Deposition of capsules on hair from SH3, SH4 and SH5

The following compositions were prepared:-
SH3 - as in Table 3 above
SH4 - which was the same as SH3 but with the omission of Jaguar C17 polymer.
SH5 - which was the same as SH3 but with polyDADMAC (poly (diallyl dimethyl ammonium chloride; Merquat 106, available from Lubrizol)) substituted for the C17 polymer (at 0.05 wt%, based on 100 % active and the total weight of the composition).

A study of the deposition of the microcapsules onto hair was carried out in the same way as described in Example 1 above.

The results are given in Table 4 below.

**Table 4: Level of deposition of microcapsules on hair, from SH3, SH4 and SH5**

| Composition | Deposition Efficiency |
|---|---|
| | (%) |
| SH3 | 23.0 |
| SH4 | 18.0 |
| SH5 | 17.8 |

It will be seen that the composition in accordance with the invention (SH3) results in higher deposition than a composition comprising a single polymer (SH4) and a composition comprising a polymer combination of the prior art (SH5).

## Claims

1. A personal cleansing composition comprising, in an aqueous continuous phase:
(i) from 5 to 30% by weight of one or more anionic cleansing surfactants;
(ii) microcapsules in which a core comprising benefit agent is encapsulated in a polymeric shell and in which the benefit agent of the core of the microcapsule (ii) is selected from perfumes,
(iii) a fragrance, and
(iv) a combination of cationic polymers comprising:
(a) at least one cationic polygalactomannan having a mean charge density at pH 7 of less than 1.2 meq per gram; and
(b) at least one cationic polygalactomannan having a mean charge density at pH 7 of at least 1.2 meq per gram.

2. A composition according to claim 1, in which the cationic polygalactomannan (a) has a mean charge density at pH 7 of from 0.5 to 1.1 meq per gram..

3. A composition according to claim 1 or claim 2, in which the cationic polygalactomannan (b) has a mean charge density at pH 7 of from 1.2 to 3 meq per gram, preferably from 1.2 to 2 meq per gram.

4. A composition according to any preceding claim, in which the polymeric shell of the microcapsule is an aminoplast resin selected from melamine glyoxal and polyurea.

5. A composition according to any preceding claim, in which the cationic polygalactomannan (a) is selected from guar hydroxypropyltrimonium chlorides having an average molecular weight in the range 800,000 to 2.5 million g/mol and a charge density ranging from 0.5 to 1.1 meq/g.

6. A composition according to any preceding claim, in which the cationic polygalactomannan (b) is selected from guar hydroxypropyltrimonium chlorides having an average molecular weight in the range 800,000 to 2.5 million g/mol and a charge density ranging from 1.2 to 2 meq/g.

7. A composition according to any preceding claim, in which the weight ratio of cationic polymer (a) to cationic polymer (b) in the composition ranges from 3:1 to 1:1.

## Patentansprüche

1. Reinigungszusammensetzung für den persönlichen Gebrauch, umfassend in einer wässrigen kontinuierlichen Phase:
(i) von 5 bis 30 Gewichts-% eines oder mehrerer anionischer Reinigungstenside;
(ii) Mikrokapseln, in denen ein Kern, umfassend ein Vorteilsmittel, in einer polymeren Hülle eingekapselt ist und worin das Vorteilsmittel des Kerns der Mikrokapsel (ii) aus Parfüms ausgewählt ist,
(iii) einen Duftstoff und
(iv) eine Kombination von kationischen Polymeren, umfassend:
(a) mindestens ein kationisches Polygalactomannan, das bei pH 7 eine mittlere Ladungsdichte von weniger als 1,2 meq pro Gramm aufweist; und
(b) mindestens ein kationisches Polygalactomannan, das bei pH 7 eine mittlere Ladungsdichte von mindestens 1,2 meq pro Gramm aufweist.

2. Zusammensetzung nach Anspruch 1, in welcher das kationische Polygalactomannan (a) bei pH 7 eine mittlere Ladungsdichte von 0,5 bis 1,1 meq pro Gramm aufweist.

3. Zusammensetzung nach Anspruch 1 oder Anspruch 2, in welcher das kationische Polygalactomannan (b) bei pH 7 eine mittlere Ladungsdichte von 1,2 bis 3 meq pro Gramm, vorzugsweise von 1,2 bis 2 meq pro Gramm, aufweist.

4. Zusammensetzung nach irgendeinem vorhergehenden Anspruch, in welcher die polymere Hülle der Mikrokapsel ein Aminoplastharz ist, ausgewählt aus Melaminglyoxal und Polyharnstoff.

5. Zusammensetzung nach irgendeinem vorhergehenden Anspruch, in welcher das kationische Polygalactomannan (a) aus Guarhydroxypropyltrimoniumchloriden ausgewählt ist, die ein durchschnittliches Molekulargewicht in dem Bereich von 800.000 bis 2,5 Millionen g/mol und eine Ladungsdichte, die von 0,5 bis 1,1 meq/g reicht, aufweisen.

6. Zusammensetzung nach irgendeinem vorhergehenden Anspruch, in welcher das kationische Polygalactomannan (b) aus Guarhydroxypropyltrimoniumchloriden ausgewählt ist, die ein durchschnittliches Molekulargewicht in dem Bereich von 800.000 bis 2,5 Millionen g/mol und eine Ladungsdichte, die von 1,2 bis 2 meq/g reicht, aufweisen.

7. Zusammensetzung nach irgendeinem vorhergehenden Anspruch, in welcher das Gewichtsverhältnis von kationischem Polymer (a) zu kationischem Polymer (b) in der Zusammensetzung von 3:1 bis 1:1 reicht.

## Revendications

1. Composition de nettoyage pour la personne comprenant, dans une phase continue aqueuse :
(i) de 5 à 30 % en masse d'un ou plusieurs tensioactifs de nettoyage anioniques ;
(ii) des microcapsules dans lesquelles un noyau comprenant un agent bénéfique est encapsulé dans une enveloppe polymère et dans lesquelles l'agent bénéfique du noyau de la microcapsule (ii) est choisi parmi des parfums,
(iii) une senteur, et
(iv) une combinaison de polymères cationiques comprenant :
(a) au moins un polygalactomannane cationique présentant une densité moyenne de charge à pH 7 inférieure à 1,2 meq par gramme ; et
(b) au moins un polygalactomannane cationique présentant une densité moyenne de charge à pH 7 d'au moins 1,2 meq par gramme.

2. Composition selon la revendication 1, dans laquelle le polygalactomannane cationique (a) présente une densité moyenne de charge à pH 7 de 0,5 à 1,1 meq par gramme.

3. Composition selon la revendication 1 ou la revendication 2, dans laquelle le polygalactomannane cationique (b) présente une densité moyenne de charge à pH 7 de 1,2 à 3 meq par gramme, de préférence de 1,2 à 2 meq par gramme

4. Composition selon l'une quelconque des revendications précédentes, dans laquelle l'enveloppe polymère de la microcapsule est une résine aminoplaste choisie parmi le mélamine glyoxal et la polyurée.

5. Composition selon l'une quelconque des revendications précédentes, dans laquelle le polygalactomannane cationique (a) est choisi parmi des chlorures de guar hydroxypropyltrimonium présentant une masse moléculaire moyenne dans l'intervalle de 800 000 à 2,5 millions g/mol et une densité de charge de 0,5 à 1,1 meq/g.

6. Composition selon l'une quelconque des revendications précédentes, dans laquelle le polygalactomannane cationique (b) est choisi parmi des chlorures de guar hydroxypropyltrimonium présentant une masse moléculaire moyenne dans l'intervalle de 800 000 à 2,5 millions g/mol et une densité de charge de 1,2 à 2 meq/g.

7. Composition selon l'une quelconque des revendications précédentes, dans laquelle le rapport massique de polymère cationique (a) à polymère cationique (b) dans la composition est de 3:1 à 1:1.
